Europäisches Patentamt

(19) European Patent Office

Office européen des brevets

(11) Veröffentlichungsnummer: **0 026 851**
.B1

(12) **EUROPÄISCHE PATENTSCHRIFT**

(45) Veröffentlichungstag der Patentschrift:
18.07.84

(51) Int. Cl.³: **A 61 K 35/80**

(21) Anmeldenummer: 80105349.7

(22) Anmeldetag: 08.09.80

(54) **Cholesterinsenkende Mittel und Verfahren zur Herstellung eines dafür geeigneten Algenextraktionsrückstandes.**

(30) Priorität: 05.10.79 DE 2940411

(43) Veröffentlichungstag der Anmeldung:
15.04.81 Patentblatt 81/15

(45) Bekanntmachung des Hinweises auf die Patenterteilung:
18.07.84 Patentblatt 84/29

(84) Benannte Vertragsstaaten:
CH FR GB LI

(56) Entgegenhaltungen:
DE - A - 1 617 948

CHEMICAL ABSTRACTS, Band 91, Nr. 17, 22. Oktober 1979, Seite 550, Zusammenfassung 139515, Columbus, Ohio, US M.A. DEVI et al.: "Hypocholesterolemic effect of diets containing algae on albino rats"
BIOLOGICAL ABSTRACTS, Band 71, Nr. 1, 1981, Zusammenfassung 4811, Philadelphia, Pennsylvania, US I. ROLLE et al.: "Cholesterol decreasing effect of the unicellular green alga Scenedesmus acutus 276-3a: 1. Effect of drum-dried algal material"
BIOLOGICAL ABSTRACTS, Band 71, Nr. 1, 1981, Zusammenfassung 4812, Philadelphia, Pennsylvania, US I. ROLLE et al.: "On the cholesterol decreasing effect of the unicellular green alga. Scenedesmus acutus 276-3a: 2 Effect of alga fractions"

(73) Patentinhaber: **Kernforschungsanlage Jülich Gesellschaft mit beschränkter Haftung, Postfach 1913, D-5170 Jülich (DE)**

(72) Erfinder: **Rolle, Ingrid, Schlagbaum 17, D-5804 Herdecke (DE)**
Erfinder: **Pabst, Wolfgang, Dr., Persebecker Strasse 40a, D-4600 Dortmund (DE)**

(56) Entgegenhaltungen: (Fortsetzung)
BIOLOGICAL ABSTRACTS, Band 70, Nr. 12, 1980, Zusammenfassung 82209 Philadelphia, Pennsylvania, US L.V. VENKATARAMAN et al.: "Toxicology and safety of algal diets in albino rats"

**Beschreibung**

Die Erfindung bezieht sich auf cholesterinsenkende Mittel auf der Basis von Fraktionierungsprodukten der einzelligen Grünalge Scenedesmus, sowie auf ein Verfahren zur Gewinnung einer für solche Mittel geeigneten Grünalgenfraktion.

Die senkende Wirkung von Meeresalgen auf den Blut-Cholesterinspiegel ist seit einiger Zeit bekannt (siehe M. H. Baslow »Marine Pharmacology«; Williams & Wilkins, Baltimore 1969). Hierüber berichten insbesondere japanische Autoren, die den Einfluß einiger Meeresalgen, die in Ostasien als Nahrungsmittel dienen, auf den Cholesterinstoffwechsel der Ratte bei diätetisch erhöhten Fett- und Cholesterinwerten untersuchten und eine erhebliche Senkung der Plasmacholesteringehalte durch Zusätze mariner Rot- und Grünalgen zur Nahrung fanden. Vereinzelt wurde auch über eine entsprechende Aktivität von Süßwasseralgen berichtet (siehe M. Okuda unter anderem, Jap. J. Nutr. 33 (1975) 3—8).

Im Handel sind in Japan Tabletten erhältlich, die Trockenpulver der einzelligen Süßwassergrünalge Chlorella enthalten und denen eine allgemein gesundheitsstabilisierende Wirkung nachgesagt wird.

Versuche zur Isolierung der in Meeresalgen enthaltenen cholesterinsenkenden Wirkstoffe haben bislang zu keinem eindeutigen Ergebnis geführt. Üblicherweise wird versucht, die wirksamen Inhaltsstoffe mit Wasser zu extrahieren oder mit organischen Lösungsmitteln im Extrakt anzureichern. Die Extraktionsrückstände wurden weitgehend vernachlässigt und als unbrauchbarer Rest verworfen.

Wirkungen, die bei systematischen Untersuchungen mit extraktionsrückständen erzielt werden konnten, wurden nicht herausgelösten, aber als löslich angesehenen Restgehalten zugeschrieben, deren Existenz auf eine nur unvollkommen arbeitende Extraktion zurückgeführt wurde (siehe T. Kaneda und K. Atai, Bull. Jap. Soc. Sci. Fish. 31 1965, 152—156).

Von M. A. Devi u. a. (siehe Nutrition Reports International, Band 20 (1), 1979, Seiten 83—90) wurde getrocknetes Algenpulver von Scenedesmus acutus als Nahrungszusatz in unterschiedlicher Menge an Ratten verfüttert und ein deutlicher cholesterinsenkender Effekt beobachtet und daraufhin eine Fraktionierung des Algenpulvers in seine Hauptbestandteile — allerdings ohne Angabe irgendwelcher Trenn- oder Aufschlußverfahren — ins Auge gefaßt.

Von den Erfindern wurde nun überraschenderweise festgestellt, daß gerade der nach wäßriger Extraktion verbleibende unlösliche Rest der mit gutem Erfolg kultivierten Süßwasser-Microalge Scenedesmus wirksamer ist als die durch die Extraktion erhaltene flüssige Phase, der bislang die Hauptaufmerksamkeit galt. Dieser Extraktionsrückstand steht in der Wirkung der Alge selbst nicht nach; er hat jedoch dieser gegenüber den Vorteil, daß begleitende, für die Aufnahme durch den Menschen lästige Farb- und Geruchsanteile weitgehend beseitigt sind und das Produkt organoleptisch neutral geworden ist.

Die erfindungsgemäßen cholesterinsenkenden Mittel der eingangs genannten Art sind demgemäß dadurch gekennzeichnet, daß die Fraktionierungsprodukte durch den nach wäßriger Extraktion zurückbleibenden Extraktionsrückstand gebildet werden.

Als besonders geeignet für die Erzeugung von cholesterinsenkenden Mitteln erweist sich der durch wäßrige Extraktion von Scenedesmus 276—3a erhaltene Extraktionsrückstand.

Vorzugsweise wird das durch Züchtung der Algen erhaltene Algentrockenpulver mit heißem Wasser von 95°C extrahiert. Ein für die orale Aufnahme besonders geeignetes Produkt ergibt sich, wenn der Heißwasserextraktion eine Behandlung mit Fettlösern vorausgeht.

Man erhält den Grünalgenextraktionsrückstand für die erfindungsgemäßen cholesterinsenkenden Mittel durch Züchtung der Alge Scenedesmus in bekannter Weise und zum Beispiel nach dem von E. Stengel in Ber. Dtsch. Bot. Ges. 83 (1970) S. 589—606 angegebenen Verfahren. Das so gewonnene Ausgangsmaterial wird dann entweder nur mit Wasser extrahiert, und zwar werden insbesondere 1 Gew.-Teil Algentrockenpulver mit 10 Gew.-Teilen Wasser von 95°C 0,5 Stunden ausgelaugt. Eine Wiederholung dieser Auslaugung durch warmes Wasser erweist sich als zweckmäßig aber nicht unbedingt notwendig. Der so ausgezogene Rückstand wird vorzugsweise gefriergetrocknet und mit üblichen Rezipienten zu Tabletten verarbeitet oder als Pulver geeigneten Speisen oder Getränken zugesetzt.

Werden die Algen vor der beschriebenen Wasserextraktion zusätzlich noch mit Fettlösern wie einer 2 : 1 Mischung von Chloroform-Methanol 24 Stunden unter Rühren bei Raumtemperatur behandelt und der abgetrennte, von Lösungsmittelresten befreite Rückstand dann mit Heißwasser wie oben extrahiert, so ist das erhaltene Endprodukt geruchs- und farbneutral und somit organoleptisch einwandfrei. Es ist für die Aufnahme durch den Menschen sehr viel besser geeignet als das zugrundeliegende Algentrockenpulver.

Die Wirksamkeit des Extraktionsrückstandes wurde durch pharmakologische Untersuchungen an Ratten getestet:

Dazu wurden 10 Versuchsgruppen von Ratten mit Futterzusätzen der nach vorstehender Beschreibung gewonnenen Extraktionsrückstände 6 Wochen ernährt. Von diesen Tieren wurden die Serum- und Lebercholesteringehalte von Cholesterinbelasteten Gruppen und Gruppen, die normale Cholesteringehalte im Futter hatten, vergleichend bestimmt.

Die Zusätze von 20% Trockenalgen beziehungsweise äquivalenten Mengen von Heißwasser — extrahierten, Lösungsmittel — extrahierten beziehungsweise Heißwasser — und Lösungsmittel — extrahierten Trockenalgen führten im Vergleich zu den Kontrolltieren, die Standardfutter mit und ohne Cholesterinzusatz erhielten zu einer deutlichen Minderung des Cholesterinanstiegs in Blut-Serum und Leber bei gleichzeitiger Senkung der Plasma-Triglyceride. Die Serumwerte cholesterinbelasteter Diätgruppen lagen um 30% die Leberwerte um 50% unter den Werten der cholesterinbelasteten Kontrollen. Der hypocholesterinämische Effekt der verwendeten Zusätze lag somit über der Wirksamkeit gebräuchlicher Pharmaka, die in der Regel eine Senkung erhöhter Plasmacholesterinwerte um 10—20% bewirken.

**Patentansprüche**

1. Cholesterinsenkende Mittel auf der Basis von Fraktionierungsprodukten der einzelligen Grünalge Scenedesmus, dadurch gekennzeichnet, daß die Fraktionierungsprodukte durch den nach wäßriger Extraktion zurückbleibenden Extraktionsrückstand gebildet werden.

2. Mittel nach Anspruch 1, dadurch gekennzeichnet, daß der Extraktionsrückstand durch Extraktion von Algentrockenpulver mit heißem Wasser erhalten worden ist.

3. Mittel nach Anspruch 1 oder 2, dadurch gekennzeichnet, daß der Algenrückstand durch zusätzliche Extraktion mit Fettlösern, insbesondere mit einer Chloroform/Methanolmischung, erhalten worden ist.

4. Mittel nach einem der Ansprüche 1 bis 3, dadurch gekennzeichnet, daß der Extraktionsrückstand von Algen vom Stamm Scenedesmus 276—3a stammt.

5. Verfahren zur Gewinnung einer für Mittel nach einem der vorangehenden Ansprüche geeigneten Grünalgenfraktion, dadurch gekennzeichnet, daß man Scenedesmus, insbesondere Scenedesmus 276—3a, in bekannter Weise züchtet und die gewonnenen Algen, insbesondere das Algentrockenpulver, mit heißem Wasser extrahiert und gegebenenfalls zusätzlich mit fettlösenden Mitteln behandelt und den Rückstand gewinnt.

6. Verfahren nach Anspruch 5, dadurch gekennzeichnet, daß die Behandlung mit fettlösenden Mitteln mittels einer Chloroform-Methanolmischung erfolgt.

7. Verfahren nach Anspruch 5 oder 6, dadurch gekennzeichnet, daß der erhaltene Extraktionsrückstand gefriergetrocknet wird.

**Claims**

1. Cholesterol-lowering composition based on fractionation products of the single-cell green alga Scendesmus, characterised in that the fractionation products are formed by the extraction residue remaining after aqueous extraction.

2. Composition according to claim 1, characterised in that the extraction residue is obtained by extraction of dried alga powder with hot water.

3. Composition according to claim 1 or 2, characterised in that the alga residue is obtained by additional extraction with fat solvent especially with a chloroform/methanol mixture.

4. Composition according to one of claims 1 to 3, characterised in that the extraction residue emanates from algae of the strain Scenedesmus 276—3a.

5. Precess for obtaining a green alga fraction suitable for a composition according to one of the preceding claims, characterised in that Scenedesmus, especially Scenedesmus 276—3a, is grown in known manner an the algae obtained, expecially the dried alga powder, are/is extracted with hot water and possibly additionally treated with fat-dissolving agents, and the residue collected.

6. Process according to claim 5, characterised in that the treatment with fat-dissolving agents is carried out by means of a chloroform-methanol mixture.

7. Process according to claim 5 or 6, characterised in that the extraction residue obtained is freeze-dried.

**Revendications**

1. Composition hypocholestérolémiante, à base de produits de fractionnement de l'algue verte unicellulaire Scenedesmus, caractérisé en ce que les produits de fractionnement sont formés par le résidu d'extraction restant après l'extraction par de l'eau.

2. Composition suivant la revendication 1, caractérisé en ce que le résidu d'extraction a été obtenu en soumettant une poudre sèche d'algue à une extraction par de l'eau chaude.

3. Composition suivant la revendication 1 ou 2, caractérisé en ce que le résidu d'algue a été obtenu par extraction supplémentaire à l'aide de solvants pour des graisses, notamment à l'aide d'un mélange de chloroforme et de méthanol.

4. Composition suivant l'une des revendications 1 à 3, caractérisé en ce que le résidu d'extraction provient d'algues de la souche Scenedesmus 276—3a.

5. Procédé d'obtention d'une fraction d'algue verte convenant pour une composition suivant l'une des revendications précédentes, caractérisé en ce qu'il consiste à cultiver, d'une manière en soi connue, Scenedesmus, notamment Scenedesmus 276—3a, et à soumettre les algues obtenues, notamment la poudre sèche d'algues, à une extraction par de l'eau chaude et, le cas échéant, en outre à un traitement par des agents dissolvant les graisses, et à recueillir le résidu.

6. Procédé suivant la revendication 5, caractérisé en ce qu'il consiste à effectuer le traitement par des agents dissolvant les graisses au moyen d'un mélange de chloroforme et de méthanol.

7. Procédé suivant la revendication 5 ou 6, caractérisé en ce qu'il consiste à lyophiliser le résidu d'extraction obtenu.